# EUROPEAN PATENT APPLICATION

(11) **EP 1 344 510 A1**
(43) Date of publication of application: **17.09.2003**
(21) Application number: 03004419.2
(22) Date of filing: 27.02.2003
(51) Int. Cl.: A61F 13/15, B05C 11/10, B05C 5/02

(54) **Method of securing elastic strands to flat substrates and products produced by the method**

(30) Priority: 15.03.2002 US 364811 P; 30.10.2002 US 283690
(71) Applicant: NORDSON CORPORATION, Westlake, Ohio 44145-1119 (US)
(72) Inventor: Hayder, Raza, Duluth, Georgia 30096 (US); Zgonc, David, Atlanta, Georgia 30319 (US)
(74) Representative: Eisenführ, Speiser & Partner

(57) **Abstract**

A method of securing an elastic strand to a sheet of material includes moving the elastic strand and the sheet in a converging manner from a position in which the elastic strand is spaced from the sheet to another position in which the elastic strand contacts one surface of the sheet. Discrete dots of adhesive are intermittently applied to the strand. The dots of adhesive secure the strand to the sheet. Various articles of manufacture may be formed using the method, including hygienic articles such as diapers.

## Description

### Cross-Reference to Related Applications

This application claims the benefit of U.S. Provisional Application No. 60/364,811 filed on March 15, 2002, and the disclosure of which is hereby incorporated by reference herein.

### Field of the Invention

The present invention generally relates to technology associated with securing elastic strands to flat substrates and, more particularly, to the securement of elastic strands to substrates such as those used in producing hygienic articles such as diapers.

### Background of the Invention

In various types of manufacturing operations, it is necessary to bond thin elastic strands to one or more sheets of material, such as woven or nonwoven materials. This practice is especially prevalent in the area of hygienic article manufacture, such as during the manufacture of diapers. Diaper manufacturing involves the application of fiberized adhesives, including temperature and/or pressure sensitive adhesives, onto flat substrates and stretched elastic strands, for example, in the areas of the waistband, leg cuffs, and standing leg gathers of the diapers. In these situations, it has been common practice to dispense continuous adhesive fibers or filaments onto either single elastic strands or multiple elastic strands at the same time, either before or after the strand has been laid against the substrate, to bond the strand(s) to the substrate(s). In this manner, overlapping portions of the same material may be bonded together with the stretched elastic strand(s) secured therebetween or two distinctly different substrates may be bonded together with the stretched elastic strand secured therebetween. This is a popular manner to elasticize specific areas of an article comprised of at least one flat substrate.

One type of adhesive dispenser which has been used extensively for bonding one or more elastic strands to one or more flat substrates is Controlled Fiberization™ (CF™ ) technology. This well known technique imparts a generally back and forth motion to a dispensed filament of adhesive in the preferred form of a swirl by impacting the filament with a plurality of jets of air. In this manner, a wider region of a substrate may be covered with adhesive dispensed from a single discharge orifice of a nozzle. The width of the adhesive pattern placed on the substrate may be widened to many times the width of the adhesive filament itself. Controlled Fiberization techniques have also been used to secure single or multiple strands of elastic to a flat substrate by dispensing the adhesive onto the strand(s) of elastic while the strand(s) are separated from the substrate. In this manner, the adhesive filament wraps itself around the strand(s) of elastic prior to joining the elastic strand(s) to the substrate thus providing a stronger bond between the elastic strands and the substrate.

Other adhesive filament dispensing techniques and apparatus have been used for producing vacillating generally sinusoidal pattern of adhesive on a substrate or, for example, a stitching pattern in which the adhesive moves back and forth generally in a sinusoidal or a zigzag motion on the substrate. Still other elastic strand securing methods include extruding a continuous layer of adhesive onto the strand after the strand has contacted the substrate. Various meltblowing techniques have also been used which essentially use randomly dispersed filaments of adhesive discharged onto one or more elastic strands either before or after the elastic strands have contacted the substrate.

Some of the main goals in this area of technology relate to achieving the necessary bond strength between the elastic strands and the substrates while at the same time transferring the desired elastic properties of the strands to the substrates. Another goal is to use as little adhesive as possible. In addition to undesirable cost increase, using too much adhesive tends to stiffen the substrate and reduce the elastic properties of the strand(s). This latter effect leads to reduced elasticity in critical areas of the diaper, such as the waistband, leg cuffs, and standing leg gathers. In addition, large fiber patterns may obstruct the communication of moisture between layers, such as between an inner layer and an absorbent outer layer.

For these and other reasons, it would be desirable to provide a method of securing one or more elastic strands to a flat substrate or sheet in a manner suitable for a high speed manufacturing environment, while also achieving the necessary bond strength, creep resistance, efficient use of adhesive, and optimization of other desired characteristics of the resulting product.

### Summary of Invention

A method of securing at least one elastic strand to a sheet of material in accordance with this invention includes moving the elastic strand and the sheet in a converging manner from a first position in which the elastic strand is spaced from the sheet to a second position in which the elastic strand is adjacent one surface of the sheet. Preferably, the elastic strand is in a stretched condition during the securement method. In the second position, the strand contacts one surface of the sheet. Discrete dots of adhesive are applied to the strand preferably by intermittently actuating a suitable adhesive dispenser such as an electrically operated valve. The intermittently applied dots of adhesive contact the strand and the sheet of material in the second position to secure the strand to the sheet. In one form of the method, the dots of adhesive are applied to the elastic strand while the elastic strand and the sheet are in the first position, that is, while the strand and the sheet are spatially separated. Alternatively or in addition, the dots of adhesive are applied to the elastic strand while the elastic strand and the sheet are in the second position, that is, while the strand contacts one surface of the sheet. The discharge orifice of the dispenser used to apply the dots of adhesive may be spaced from the elastic strand or it may contact the elastic strand. Also, the discharge orifice may be of any desired shape including, for example, circular or square shapes. Preferably, when the adhesive dots are applied at the first position, the adhesive discharge orifice is spaced from the elastic strand and when the adhesive dots are applied at the second position, the discharge orifice is in contact with the elastic strand.

In another method performed in accordance with the inventive principles, a slot nozzle is used having an adhesive discharge slot with a length and a width. The length is greater than the width and the adhesive discharge slot may be oriented adjacent the elastic strand with the strand moving parallel to the length of the discharge slot or transverse to the length of the discharge slot. Discrete dots of adhesive are then intermittently applied to the strand from the discharge slot. In this embodiment, the dots of adhesive will typically have a more elongate shape and the lengthwise dimension of the dot may be oriented parallel or transverse to the strand. In accordance with this aspect of the invention, the discrete dots of adhesive may be applied to the elastic strand in any of the manners described above. A strand guide slot may be used directly adjacent each discharge slot to ensure that the strand is stabilized against any significant movement transverse to the direction of its travel through the slot. The strand is preferably spaced slightly from the adhesive discharge opening of the slot.

Any of the methods discussed generally above may be applied to situations involving the securement of more than one elastic strand to a sheet of material. That is, multiple spaced apart elastic strands are used in the manufacture of various articles, such as in diaper manufacture, to form elasticized sections of the article. In these situations, a plurality of discharge orifices, which may be round orifices or elongate slots, are positioned adjacent a corresponding plurality of elastic strands. Discrete dots of adhesive are then intermittently applied in essentially parallel lines along each of the respective elastic strands in any of the manners previously described.

The invention further contemplates articles formed from flat substrates with at least one elastic strand secured on at least one substrate and, preferably, between two substrates. The elastic strand, and the first and second substrates are secured together by a plurality of discrete dots of adhesive extending along the elastic strand between the first and second flat substrates. It will be appreciated that the first and second flat substrates may either be completely separate materials secured together or may be portions of the same material which have been folded over to form the first and second substrates with the elastic strand(s) held therebetween. It will also be appreciated that various articles may be manufactured in accordance with the invention including hygienic articles, such as diapers, or other articles formed of flat substrates with elasticized portions.

The present invention generally provides superior process control in elastic strand securing applications. The elastic strands will be effectively coated with dots of adhesive at a desired frequency. The dots of adhesive will form localized areas characterized by high bond strength between the elastic strand(s) and the substrate. At the same time, the elastic properties of the strands will not be significantly compromised and this should provide for better creep resistance or retained elasticity in the final product. Since the adhesive attachment method of this invention provides for increased creep resistance or retained elasticity in the final product, it is possible to use finer denier elastic strands. The invention also enables the use of less adhesive. Each of these aspects of the invention results in reducing the costs associated with manufacturing the product. A further increase in creep resistance should be obtainable if the adhesive dots are completely separated, i.e., there is no adhesive tailing between dots. Low adhesive add-on rates achieved by the invention also lead to enhanced softness of the manufactured article, and less bleedthrough of adhesive through the substrate(s). This latter advantage allows the use of lower gauge substrates leading to further cost reductions. Moreover, available technology in the form of bead dispensing nozzles or slot nozzles may be used and this will lead to lower costs and production downtime.

Although preferred embodiments are discussed in detail herein, it will be appreciated that the adhesive dispensing methods of this invention may be applied with the adhesive nozzle spaced from the elastic strand or in contact with the elastic strand. Also, the elastic strand may be spaced from the sheet or substrate or it may be in contact with the sheet or substrate when receiving the adhesive. Any combination of the above may be employed, using orifices or slots of any desired shape and orientation with respect to the strand, depending on the needs of the application.

These and other features, objects and advantages of the invention will be more readily apparent to those of ordinary skill in the art upon review of the following detailed description of the preferred embodiments, taken in conjunction with the accompanying drawings.

### Brief Description of Drawings

Fig. 1 is a schematic elevational view illustrating a method for intermittently applying adhesive dots to one or more elastic strands prior to joining the elastic strands with a flat substrate.
Fig. 2 is a schematic view similar to Fig. 1, but illustrating a method of intermittently applying dots of adhesive after the elastic strand has contacted one surface of the flat substrate.
Fig. 3 is a top view illustrating the preferred adhesive pattern of intermittent, discrete and separated dots of adhesive on multiple elastic strands.
Fig. 4 is a fragmented front elevational view showing the nozzle portion of a slot nozzle dispenser for intermittently applying dots of adhesive onto three elastic strands.
Fig. 5 is a bottom view of the slot nozzle shown in Fig. 4, but with the strand guide removed for clarity.
Fig. 6 is a cross sectional view taken along lines 6-6 of Fig. 4 illustrating the method of applying discrete dots of adhesive onto the moving elastic strand.

### Detailed Description of the Preferred Embodiments

For purposes of this description, words of direction such as "upward", "vertical", "horizontal", "right", "left" and the like are applied in conjunction with the drawings for purposes of clarity in the present description only. As is well known, liquid dispensing devices may be oriented in substantially any orientation, so these directional words should not be used to imply any particular absolute directions for an apparatus consistent with the invention.

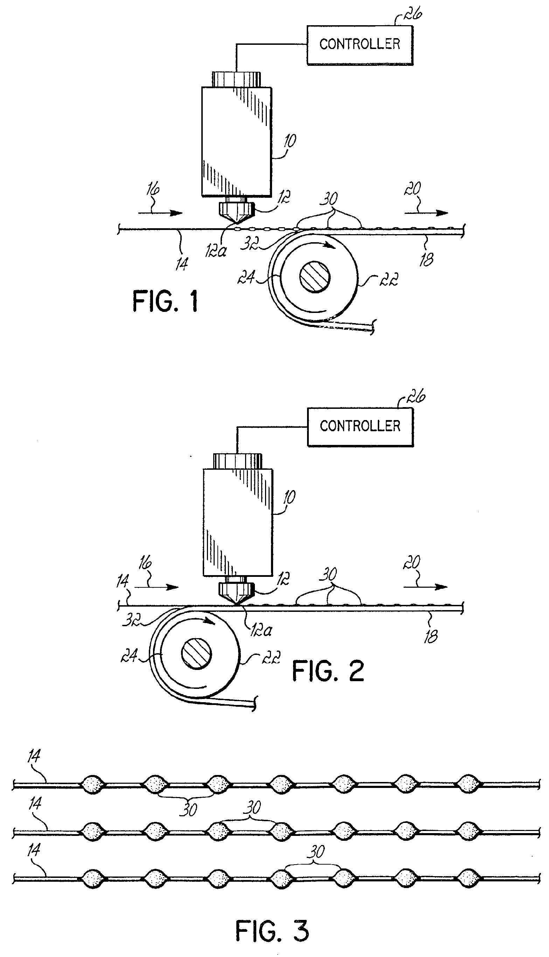

Fig. 1 illustrates one embodiment of the method of this invention which uses an adhesive dispenser 10 including a nozzle 12. Nozzle 12 may include a circular discharge orifice, a more elongate slot-shaped orifice, or other types of orifices suitable for dispensing discrete dots of adhesive. In this embodiment, one or more stretched Lycra strands 14 are moving in the direction of arrow 16 and a flat sheet 18 of substrate material, such as a woven or nonwoven material, is moving in the direction of arrow 20 around a conventional guide cylinder 22 rotating in the direction of arrow 24. Strand 14 is stretched so that, upon attachment to sheet 18, the sheet 18 will be elasticized generally along a line defined by strand 14. Dispenser 10 is operated by a suitable controller 26 for intermittently actuating a valve (not shown) within the dispenser 10. The valve actuates on and off to selectively allow and prevent the flow of adhesive through the nozzle 12. The apex or tip 12a of the nozzle 12 is spaced a short distance from the Lycra strand 14 and accurately dispenses adhesive dots 30 directly onto the strand 14 immediately prior to or upstream from the point 32 where the strand 14 meets the substrate 18. Thus, during the time that it takes for the strand 14 to reach point 32, each adhesive dot 30 will flow around all sides of the strand 14 including the lower side (as viewed in Fig. 1) to ensure full bonding between the strand 14 and the upper surface of the substrate 18.

The frequency of the adhesive dots 30 will depend on the application conditions and needs, however, in one exemplary embodiment, there may be 4-7 dots 30 per inch in a diaper manufacturing application. The adhesive discharge orifice of the nozzle 12 may be circular and generally about 0.010 inch to about 0.020 inch in diameter when a pressure sensitive hot melt adhesive having a viscosity of 1000-4000 centipoise is used at a temperature of 275° F to 350° F and a pressure of 400 psi to 800 psi. Of course, many other specific parameters for either the dispenser 10 or nozzle 12, or both, as well as for the adhesive, may be used depending on the application. The distance from the discharge orifice at the apex 12a of the nozzle 12 to the elastic strand 14 may be from about 0.010 inch to about 0.5 inch. Again, however, this distance may also change depending on the needs of the application. One preferred dispenser is the ES400 dispenser available from Nordson Corporation of Westlake, Ohio which uses an electromagnetic coil capable of cycling at a high frequency. As will be appreciated, higher speed production lines will require a higher frequency actuation to achieve the desired spacing of adhesive dots 30 on the elastic strands 14.

Fig. 2 illustrates another method performed in accordance with the inventive principles. In this method, the dispenser 10 and, more particularly, the discharge nozzle 12 is positioned upstream of the point 32 of joinder between strand 14 and substrate 18 such that the adhesive dots are dispensed after the Lycra strand(s) 14 have contacted the flat substrate 18. Also, this embodiment illustrates the option of contacting the strand 14 with the apex 12a of the nozzle 12 while intermittently dispensing the dots 30 of adhesive. In this case, the dots 30 of adhesive are dispensed onto the Lycra strand(s) and the upper surface of the flat substrate 18 in a simultaneous fashion. This, as in the first embodiment, forms discrete bond points between the elastic strand(s) 14 and the mating surface of the substrate 18.

In the embodiments of Figs. 1 and 2, it will be appreciated that nozzles 12 may be used which dispense only through one discharge orifice or, alternatively, through multiple discharge orifices. In the latter case, for example, each orifice would be positioned adjacent to a corresponding elastic strand 14 such that discrete, separated dots of adhesive 30 would be dispensed onto a corresponding number of elastic strands 14 as, for example, illustrated in the top view of Fig. 3. Alternatively, in the various embodiments of the invention there may be thinner areas of adhesive between adjacent dots 30. For example, this may occur if dispenser 10 is cycled "on" and "off" in such a manner that an adhesive tail is formed between each dot. That is, dispenser 10 might not fully shut off between adhesive dot dispensing cycles. The shape of the adhesive dots 30 themselves may also take different forms. The dots 30 may be essentially circular in shape as shown in Fig. 3, or the dots 30 may be more elongated in directions parallel or transverse to the length of the elastic strand(s) 14. This is the case, for example, if a slot nozzle is used with the length of the slot extending parallel or transverse to the length of the elastic strand(s) 14 as discussed below.

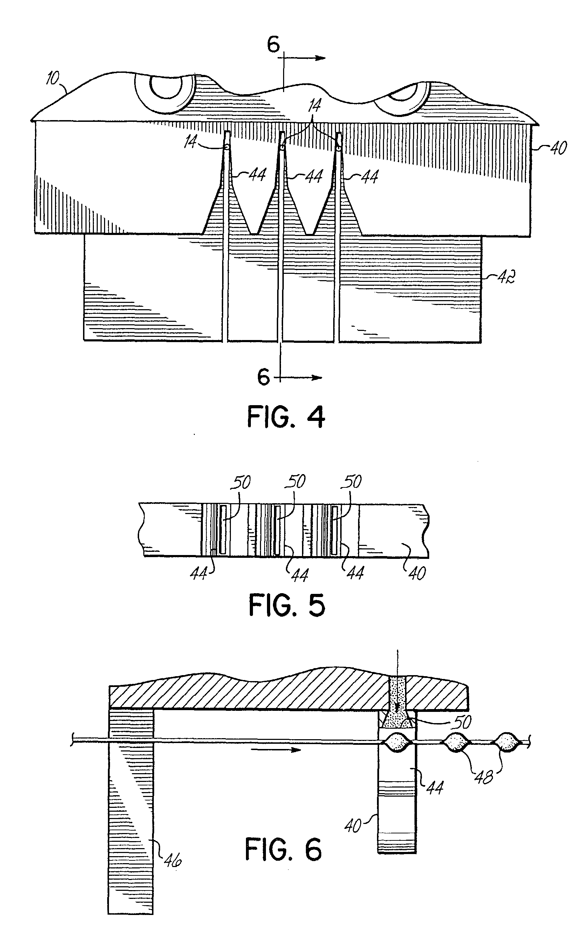

Figs. 4-6 illustrate another embodiment of the invention utilizing an illustrative slot nozzle 40 including an elastic strand guide 42 for ensuring that the strands 14 are held steady as they move through the respective slots 44 of the nozzle 40. Each strand 14 moves through a slot 46 in the guide 42 and then through a slot 40 in the adhesive discharge nozzle. An intermittently actuated valve (not shown) may be used as discussed in connection with the first embodiment to discharge elongate dots 48 of adhesive onto each strand from openings 50 associated with the respective discharge slots 40. In this embodiment, the strands 14 are again preferably spaced from the discharge openings 50 as in the first embodiment by a spacing that may be determined according to the application needs. The length of the opening 50 may be also determined by application needs. The chart below illustrates one preferred set of dimensions for the strand size when, for example, Lycra is used, and the corresponding recommended minimum slot width and discharge orifice width.

| Comparison Between Elastic Strand Size and Nozzle Dimensions | | |
|---|---|---|
| **Elastic Strand (Denier)** | **Slot Width** | **Discharge Orifice Width** |
| 420 mm | 0.23 mm | 0.20 mm |
| 490 mm | 0.25 mm | 0.23 mm |
| 560 mm | 0.28 mm | 0.25 mm |
| 700 mm | 0.32 mm | 0.30 mm |
| 840 mm | 0.35 mm | 0.30 mm |
| 1120 mm | 0.47 mm | 0.42 mm |

The dispenser in this embodiment may be the same as dispenser 10 of the first embodiment. As shown in the figures, slot opening 50 may be oriented with its length extending parallel to the corresponding strand 14. As another alternative, slot opening 50 may be oriented with its lengthwise dimension extending transverse to or across the direction that strand 14 moves. This may better allow the adhesive to flow around the sides of the strand 14.

In accordance with the invention, one or more elastic strands are coated with discrete, separated dots of adhesive either before and/or after the strand contacts one surface of the sheet. As the elastic is stretched when applied and bonded to the sheet, the sheet is elasticized along the region of the strand or strands. In addition to contacting the sheet, it will be appreciated that the adhesive should contact enough of the strand to form a sufficient bond between the sheet and the strand. This may involve fully coating all sides of the strand or only partially coating the strand. In the embodiments described above, an additional linear formation of adhesive, such as one applied by a meltblown nozzle, may be applied to the sheet, prior to contact with the strand, to assist with the bond. Dots of adhesive may be applied from only one side of the strand, such as the upper side, or from opposite sides of the strand, such as upper and lower sides. If applied from opposite sides of the strand, the dots may be applied from directly opposite locations such that they contact the same point along the strand, or they may be applied in a staggered format with upper and lower dots of adhesive alternating along the strand. In this manner, it may be possible to apply more dots of adhesive per inch. The number of adhesive dispensers on either or both sides of the strand may be varied according to the needs of the application.

While the present invention has been illustrated by a description of preferred embodiments and while these embodiments have been described in some detail, it is not the intention of the Applicants to restrict or in any way limit the scope of the appended claims to such detail. Additional advantages and modifications will readily appear to those skilled in the art. The various features of the invention may be used alone or in numerous combinations depending on the needs and preferences of the user. This has been a description of the present invention, along with the preferred methods of practicing the present invention as currently known. However, the invention itself should only be defined by the appended claims, wherein we claim:

It follows a list of further embodiments of the invention:
Embodiment 1. A method of intermittently applying discrete dots of adhesive to an elastic strand for securing the elastic strand to a sheet of material comprising:
   moving the elastic strand and the sheet in a converging manner from a first position in which the elastic strand is spaced from the sheet to a second position in which the elastic strand contacts one surface of the sheet,
   intermittently applying discrete dots of adhesive to the strand, and
   contacting the dots of adhesive, the strand and the sheet with one another when the elastic strand and the sheet are in the second position to secure the strand to the sheet.
Embodiment 2. The method of with the features of embodiment 1, wherein the step of intermittently applying the discrete dots of adhesive further comprises:
   applying the dots of adhesive to the elastic strand while the elastic strand and the sheet are in the first position.
Embodiment 3. The method of with the features of embodiment 1, wherein the step of intermittently applying the discrete dots of adhesive further comprises:
   applying the dots of adhesive to the elastic strand while the elastic strand and the sheet are in the second position.
Embodiment 4. The method of with the features of embodiment 2, wherein the adhesive is applied from a nozzle having an adhesive discharge orifice and the method further comprises:
   spacing the adhesive discharge orifice from the elastic strand while applying the dots of adhesive to the elastic strand.
Embodiment 5. The method of with the features of embodiment 3, wherein the adhesive is applied from a nozzle having an adhesive discharge orifice and the method further comprises:
   spacing the adhesive discharge orifice from the elastic strand while applying the dots of adhesive to the elastic strand.
Embodiment 6. The method of with the features of embodiment 2, wherein the adhesive is applied from a nozzle having an apex portion with an adhesive discharge orifice and the method further comprises:
   contacting the apex portion with the elastic strand while applying the dots of adhesive to the elastic strand.
Embodiment 7. The method of with the features of embodiment 3, wherein the adhesive is applied from a nozzle having an apex portion with an adhesive discharge orifice and the method further comprises:
   contacting the apex portion with the elastic strand while applying the dots of adhesive to the elastic strand.
Embodiment 8. The method of with the features of embodiment 1, wherein the adhesive is applied from a nozzle having an adhesive discharge slot, the slot having a length and a width, with the length being greater than the width, and the method further comprises:
   orienting the adhesive discharge slot adjacent the elastic strand, and
   intermittently applying the discrete dots of adhesive to the strand from the discharge slot.
Embodiment 9. The method of with the features of embodiment 8, wherein orienting the adhesive discharge slot further comprises orienting the length parallel to the strand.
Embodiment 10. The method of with the features of embodiment 8, wherein orienting the adhesive discharge slot further comprises orienting the length transverse to the strand.
Embodiment 11. The method of with the features of embodiment 8, wherein the step of intermittently applying the discrete dots of adhesive further comprises:
   applying the dots of adhesive to the elastic strand while the elastic strand and the sheet are in the first position.
Embodiment 12. The method of with the features of embodiment 8, wherein the step of intermittently applying the discrete dots of adhesive further comprises:
   applying the dots of adhesive to the elastic strand while the elastic strand and the sheet are in the second position.
Embodiment 13. The method of with the features of embodiment 8 further comprising:
   spacing the adhesive discharge slot from the elastic strand while applying the dots of adhesive to the elastic strand.
Embodiment 14. The method of with the features of embodiment 8 further comprising:
   contacting the adhesive discharge slot with the elastic strand while applying the dots of adhesive to the elastic strand.
Embodiment 15. A method of intermittently applying discrete dots of adhesive to a plurality of spaced apart elastic strands for securing the plurality of spaced apart elastic strands to a sheet of material comprising:
   moving the elastic strands and the sheet in a converging manner from a first position in which the elastic strands are spaced from the sheet to a second position in which the elastic strands contact one surface of the sheet,
   intermittently applying discrete dots of adhesive to each of the strands, and
   contacting the dots of adhesive, the strands and the sheet with one another when the elastic strands and the sheet are in the second position to secure the strands to the sheet.
Embodiment 16. The method of with the features of embodiment 15, wherein the step of intermittently applying the discrete dots of adhesive further comprises:
   applying the dots of adhesive to the elastic strands while the elastic strands and the sheet are in the first position.
Embodiment 17. The method of with the features of embodiment 15, wherein the step of intermittently applying the discrete dots of adhesive further comprises:
   applying the dots of adhesive to the elastic strands while the elastic strands and the sheet are in the second position.
Embodiment 18. The method of with the feature of embodiment 16, wherein the adhesive is applied from a nozzle having a plurality of adhesive discharge orifices corresponding to the plurality of elastic strands and the method further comprises:
   spacing the adhesive discharge orifices from the respective elastic strands while applying the dots of adhesive to the elastic strands.
Embodiment 19. The method of with the features of embodiment 17, wherein the adhesive is applied from a nozzle having a plurality of-adhesive discharge orifices corresponding to the plurality of elastic strands and the method further comprises:
   spacing the adhesive discharge orifices from the respective elastic strands while applying the dots of adhesive to the elastic strands.
Embodiment 20. The method of with the features of embodiment 15, wherein the adhesive is applied from a nozzle having an apex portion with a plurality of adhesive discharge orifices corresponding to the plurality of elastic strands and the method further comprises:
   contacting the apex portion with the elastic strands while applying the dots of adhesive to the elastic strands.
Embodiment 21. The method of with the features of embodiment 15, wherein the adhesive is applied from a nozzle having a plurality of adhesive discharge slots corresponding to the plurality of elastic strands, each slot having a length and a width, with the length being greater than the width, and the method further comprises:
   orienting the adhesive discharge slots respectively adjacent the elastic strands, and
   intermittently applying the discrete dots of adhesive to the strands from the discharge slots.
Embodiment 22. The method of with the features of embodiment 21, wherein orienting the adhesive discharge slots further comprises orienting the length of each slot parallel to the corresponding strand.
Embodiment 23. The method of with the features of embodiment 21, wherein orienting the adhesive discharge slots further comprises orienting the length of each slot transverse to the corresponding strand.
Embodiment 24. The method of with the features of embodiment 21, wherein the step of intermittently applying the discrete dots of adhesive further comprises:
   applying the dots of adhesive to the elastic strands while the elastic strands and the sheet are in the first position.
Embodiment 25. The method of with the features of embodiment 21, wherein the step of intermittently applying the discrete dots of adhesive further comprises:
   applying the dots of adhesive to the respective elastic strands while the elastic strands and the sheet are in the second position.
Embodiment 26. The method of with the features of embodiment 24 further comprising:
   spacing the adhesive discharge slots from the respective elastic strands while applying the dots of adhesive to the elastic strands.
Embodiment 27. The method of with the features of embodiment 25 further comprising:
   spacing the adhesive discharge slots from the respective elastic strands while applying the dots of adhesive to the elastic strands.
Embodiment 28. An article comprising:
   a first flat substrate, and
   an elastic strand secured on said first flat substrate by a plurality of discrete, separated dots of adhesive contacting said elastic strand and said first flat substrate.
Embodiment 29. The article of with the features of embodiment 28 further comprising:
   a plurality of elastic strands secured on said first flat substrate by a plurality of discrete dots of adhesive respectively contacting said elastic strand and said first flat substrate.
Embodiment 30. The article of with the features of embodiment 29 further comprising:
   a second flat substrate secured on an opposite side of said elastic strands from said first flat substrate.
Embodiment 31. The article of with the features of embodiment 28 further comprising:
   a second flat substrate secured on an opposite side of said elastic strand from said first flat substrate.

## Claims

1. A method of intermittently applying discrete dots of adhesive to an elastic strand for securing the elastic strand to a sheet of material comprising:
moving the elastic strand and the sheet in a converging manner from a first position in which the elastic strand is spaced from the sheet to a second position in which the elastic strand contacts one surface of the sheet,
intermittently applying discrete dots of adhesive to the strand,
and
contacting the dots of adhesive, the strand and the sheet with one another when the elastic strand and the sheet are in the second position to secure the strand to the sheet.

2. The method of claim 1, wherein the step of intermittently applying the discrete dots of adhesive further comprises:
applying the dots of adhesive to the elastic strand while the elastic strand and the sheet are in the first position.

3. The method of claim 1, wherein the step of intermittently applying the discrete dots of adhesive further comprises:
applying the dots of adhesive to the elastic strand while the elastic strand and the sheet are in the second position.

4. The method of claim 2, wherein the adhesive is applied from a nozzle having an adhesive discharge orifice and the method further comprises:
spacing the adhesive discharge orifice from the elastic strand 5 while applying the dots of adhesive to the elastic strand.

5. The method of claim 3, wherein the adhesive is applied from a nozzle having an adhesive discharge orifice and the method further comprises:
spacing the adhesive discharge orifice from the elastic strand while applying the dots of adhesive to the elastic strand.

6. The method of claim 2, wherein the adhesive is applied from a nozzle having an apex portion with an adhesive discharge orifice and the method further comprises:
contacting the apex portion with the elastic strand while applying the dots of adhesive to the elastic strand.

7. The method of claim 3, wherein the adhesive is applied from a 20 nozzle having an apex portion with an adhesive discharge orifice and the method further comprises:
contacting the apex portion with the elastic strand while applying the dots of adhesive to the elastic strand.

8. The method of claim 1 , wherein the adhesive is applied from a nozzle having an adhesive discharge slot, the slot having a length and a width, with the length being greater than the width, and the method further comprises:
orienting the adhesive discharge slot adjacent the elastic strand, and
intermittently applying the discrete dots of adhesive to the strand from the discharge slot.

9. The method of claim 8, wherein orienting the adhesive discharge slot further comprises orienting the length parallel to the strand.

10. The method of claim 8, wherein orienting the adhesive discharge slot further comprises orienting the length transverse to the 15 strand.

11. The method of claim 8, wherein the step of intermittently applying the discrete dots of adhesive further comprises:
applying the dots of adhesive to the elastic strand while the 20 elastic
strand and the sheet are in the first position.

12. The method of claim 8, wherein the step of intermittently applying the discrete dots of adhesive further comprises:
applying the dots of adhesive to the elastic strand while the elastic strand and the sheet are in the second position.

13. The method of claim 8 further comprising:
spacing the adhesive discharge slot from the elastic strand while applying the dots of adhesive to the elastic strand.

14. A method of intermittently applying discrete dots of adhesive to a plurality of spaced apart elastic strands for securing the plurality of spaced apart elastic strands to a sheet of material comprising:
moving the elastic strands and the sheet in a converging manner from a first position in which the elastic strands are spaced from the sheet to a second position in which the elastic strands contact one surface of the sheet,
intermittently applying discrete dots of adhesive to each of the strands, and
contacting the dots of adhesive, the strands and the sheet with one another when the elastic strands and the sheet are in the second position to secure the strands to the sheet,
wherein the adhesive is applied from a nozzle having a plurality of adhesive discharge slots corresponding to the plurality of elastic strands, each slot having a length and a width, with the length being greater than the width, and the method further comprises:
orienting the adhesive discharge slots respectively adjacent the elastic strands, and
intermittently applying the discrete dots of adhesive to the strands from the discharge slots,
wherein the step of intermittently applying the discrete dots of adhesive further comprises:
applying the dots of adhesive to the elastic strands while the elastic strands and the sheet are in the first position.

15. A method of intermittently applying discrete dots of adhesive to a plurality of spaced apart elastic strands for securing the plurality of spaced apart elastic strands to a sheet of material comprising:
moving the elastic strands and the sheet in a converging manner from a first position in which the elastic strands are spaced from the sheet to a second position in which the elastic strands contact one surface of the sheet,
intermittently applying discrete dots of adhesive to each of the strands, and
contacting the dots of adhesive, the strands and the sheet with one another when the elastic strands and the sheet are in the second position to secure the strands to the sheet,
wherein the adhesive is applied form a nozzle having a plurality of adhesive discharge slots corresponding to the plurality of elastic strands, each slot having a length and a width, with the length being greater than the width, and the method further comprises:
orienting the adhesive discharge slots respectively adjacent the elastic strands, and
intermittently applying the discrete dots of adhesive to the strands form the discharge slots,
wherein the step of intermittently applying the discrete dots of adhesive further comprises:
applying the dots of adhesive to the respective elastic strands while the elastic strands and the sheet are in the second position.
